# EUROPEAN PATENT APPLICATION

(11) **EP 4 718 396 A1**
(43) Date of publication of application: **01.04.2026**
(21) Application number: 25193152.3
(22) Date of filing: 31.07.2025
(51) Int. Cl.: G06V 20/69

(54) **METHOD OF DETECTING CELL-BY-CELL STAINING INTENSITY BASED ON STAINING INTENSITY DETECTION MODEL**

(30) Priority: 26.09.2024 KR 20240130430
(71) Applicant: Aivis Inc., Seoul 06221 (KR)
(72) Inventor: LEE, Dae Hong, 07318 Seoul (KR); FAN, Ming, 10418 Goyang-si, Gyeonggi-do (KR); JANG, Sung Yong, 11699 Uijeongbu-si, Gyeonggi-do (KR); CHOI, Soo Hwan, 05268 Seoul (KR)
(74) Representative: Isarpatent

(57) **Abstract**

According to one embodiment of the present disclosure, a method of detecting cell-by-cell staining intensity from a pathological image composed of a tissue slide image may include: extracting a bounding box including a detection target cell from the tissue slide image; inferring a staining intensity class of the bounding box based on a trained staining intensity detection model; and displaying bounding boxes inferred to be different staining intensity classes in different ways.

## Description

### CROSS-REFERENCE TO RELATED APPLICATION

This application claims priority to and the benefit of Korean Patent Application No. 2024-0130430, filed on September 26, 2024, the disclosure of which is incorporated herein by reference in its entirety.

### BACKGROUND

### 1. Field of the Invention

The technical idea of the present disclosure relates to a method of detecting cell-by-cell staining intensity, and more specifically, to a method for detecting cell-by-cell staining intensity per cell based on a staining intensity detection model.

### 2. Discussion of Related Art

Traditional pathological diagnostic methods use a method of placing a body tissue or cellular sample on a glass slide and observing it under a microscope. This method requires visual inspection and analysis of each cell, which is time-consuming, and the process of obtaining test results may be complicated and delayed. In particular, as the number of patients with cancer rapidly increases along with the increase in the elderly population, it is becoming difficult to efficiently meet the demand for pathological diagnostics with this traditional method.

The introduction of digital pathology is emerging as an alternative to solve these problems. Digital pathology is a method of scanning a slide sample, converting it into a digital image, and managing and analyzing it through a computer. This method can significantly improve the efficiency of pathological diagnosis through automated analysis and minimize delays in patient treatment by increasing test speed. However, in order for digital pathology to operate effectively, technological infrastructure that enables efficiently processing and transmission of large amounts of digital image data is required.

In response to this need, recent research and technology development are being conducted to seek various methods of improving digital pathology data processing and analysis. In particular, technologies are being developed to provide analytical results of digital images in real time through efficient data communication between servers and user terminals and to easily visualize them through user interfaces. Through this, a foundation is being formed to further increase the accuracy and speed of digital pathology and improve the overall quality of pathological diagnosis.

For example, in these digital pathology analyses, stained slides are used to confirm the expression of specific proteins such as human epidermal growth factor receptor 2 (HER2). HER2 is a protein that is overexpressed in certain types of cancer such as breast cancer, and accurately identifying the presence and intensity of this protein is critical in determining the direction of treatment.

In digital pathology analysis, diagnosis is carried out using techniques such as immunohistochemistry (IHC) to determine the presence and intensity of the HER2 protein. For example, to confirm the expression of the HER2 protein, a slide is stained using a specific antibody, and the stained sample is analyzed in a digital pathology system.

Even when the same slide of the stained sample is viewed, the interpretation may vary from pathologist to pathologist, and especially when the staining intensity is slightly different, the diagnosis results may not be consistent. This subjectivity may interfere with accurate diagnosis and treatment planning.

### SUMMARY OF THE INVENTION

The problem to be solved by the technical idea of the present disclosure is to provide a method of analyzing a tissue image by providing staining intensities classified into a plurality of classes for each cell.

According to one embodiment of the present disclosure, a method of detecting cell-by-cell staining intensity from a pathological image composed of a tissue slide image may include: extracting a bounding box including a detection target cell from the tissue slide image; inferring a staining intensity class of the bounding box based on a trained staining intensity detection model; and displaying the bounding boxes inferred to be different staining intensity classes in different ways.

According to one embodiment, the staining intensity classes may be classified into a negative tumor cell class, a weak tumor cell class, a moderate tumor cell class, and a strong tumor cell class.

According to one embodiment, the method may include training the staining intensity detection model based on a training data set labeled with one of a plurality of staining intensity classes for the bounding box.

According to one embodiment, the staining intensity detection model may be trained based on a training data set labeled as a non-tumor cell whose bounding box is not included in the staining intensity class.

According to one embodiment, inferring the staining intensity class may include: calculating a confidence value for each staining intensity class for the bounding box; and selecting one of the staining intensity classes among the plurality of staining intensity classes based on the calculated confidence value.

According to one embodiment, the confidence value may have a bias that is adjustable by a user for each pair of the staining intensity classes, and when at least one bias is adjusted, reselection of the staining intensity class may be performed.

According to one embodiment, the staining intensity detection model may be a model utilizing a self-supervised learning-based feature extraction model as a backbone network.

### BRIEF DESCRIPTION OF THE DRAWINGS

The above and other objects, features and advantages of the present invention will become more apparent to those of ordinary skill in the art by describing exemplary embodiments thereof in detail with reference to the accompanying drawings, in which:
FIG. 1 shows a diagram illustrating components of a staining intensity detection device according to one embodiment of the present disclosure;
FIG. 2 shows a diagram illustrating quantitative results calculated according to one embodiment;
FIG. 3 shows a diagram illustrating an example of detecting cell-by-cell staining intensity according to a previous embodiment;
FIG. 4 shows a flowchart illustrating a staining intensity detection method according to one embodiment;
FIGS. 5A and 5B show diagrams illustrating types of training data sets to be input to a staining intensity detection model according to one embodiment;
FIG. 6 shows a diagram illustrating a structure of a staining intensity detection model according to one embodiment;
FIG. 7 shows a diagram illustrating a user interface/user experience (UI/UX) capable of adjusting a bias of a confidence value according to one embodiment; and
FIG. 8 shows a diagram illustrating an example of displaying cell-by-cell staining intensity according to staining intensity class according to one embodiment.

### DETAILED DESCRIPTION OF EXEMPLARY EMBODIMENTS

Hereinafter, various embodiments of the present disclosure will be described in connection with the accompanying drawings. Various embodiments of the present disclosure may have various modifications and various embodiments, and thus specific embodiments are illustrated in the drawings and detailed descriptions related thereto are described. However, this is not intended to limit various embodiments of the present disclosure to specific embodiments but should be understood to include all modifications and/or equivalents or substitutes included in the spirit and technical scope of various embodiments of the present disclosure. In connection with the description of the drawings, similar reference numerals are used for similar components.

In various embodiments of the present disclosure, terms such as "comprise" or "have" are intended to specify the presence of features, numbers, steps, operations, components, or combinations thereof described herein and should not be understood as precluding the possibility of the presence or addition of one or more other features, numbers, steps, operations, components, parts, or combinations thereof.

In various embodiments of the present disclosure, the term "or" and the like include any and all combinations of the words listed together. For example, "A or B" may include A, may include B, or may include both A and B.

The terms "first," "second," and the like used in various embodiments of the present disclosure may modify various components of the various embodiments, but they do not limit the components. For example, these terms do not limit the order and/or importance of the components and may be used to distinguish one component from another.

When it is mentioned that a component is "connected" or "linked" to another component, it should be understood that the component may be directly connected or linked to the other component but that new other components may also be present between the component and the other component.

In the embodiments of the present disclosure, terms such as "module," "unit," "part," and the like are terms used to refer to components that perform at least one function or operation, and these components may be implemented as hardware or software, or as a combination of hardware and software. In addition, a plurality of "modules," "units," "parts," and the like are may be integrated into at least one module or chip and implemented as at least one processor, except in cases in which each of them needs to be implemented as individual specific hardware.

Terms, such as those defined in commonly used dictionaries, should be interpreted as having a meaning that is consistent with their meaning in the context of the relevant art and are not interpreted in an idealized or overly formal sense unless explicitly defined in various embodiments of the present disclosure.

Hereinafter, embodiments of the present disclosure will be described in detail with reference to the attached drawings.

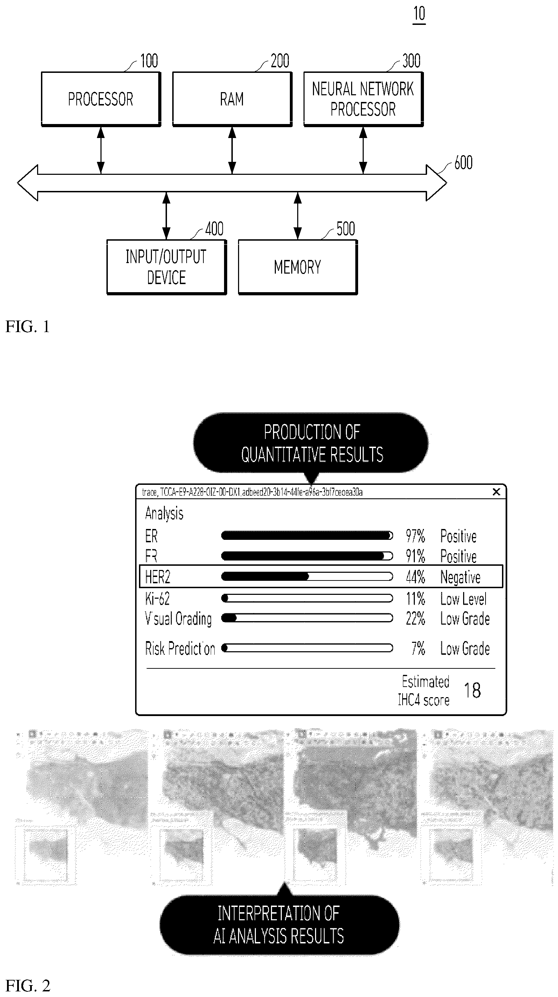

FIG. 1 shows a diagram illustrating components of a staining intensity detection device according to one embodiment of the present disclosure.

Referring to FIG. 1, the staining intensity detection device 10 may analyze an input image based on a neural network to detect cell-by-cell staining intensity. At this time, the input image may be an image in which tissue cells are stained, and it may be referred to as a pathological image, a whole slide image (WSI), or a stained tissue slide image. An image in which tissue cells are stained may include an image in which human epidermal growth factor receptor 2 (HER2), estrogen receptor (ER), progesterone receptor (PR) are stained through immunohistochemical (IHC) staining and a hematoxylin and eosin (H&E) stained image, and it may include all types of images in which a specific type of cell is stained and identifiable in a pathological image.

The staining intensity detection device 10 may include any type of device on which calculations may be performed by a processor 100 and may be applied to a smart TV, a smart phone, a mobile device, a video display device, a measuring device, an internet of things (IoT) device, and it may also be mounted on one of various types of electronic devices.

The staining intensity detection device 10 may include at least one internet protocol (IP) block and a neural network processor 300. The staining intensity detection device 10 may include various types of IP blocks, and for example, as illustrated in FIG. 1, the staining intensity detection device 10 may include IP blocks such as a processor 100, a random access memory (RAM) 200, an input/output device 400, and a memory 500. In addition, the staining intensity detection device 10 may further include other general-purpose components such as a multi-format codec (MFC), a video module (e.g., a camera interface, a Joint Photographic Experts Group (JPEG) processor, a video processor, a mixer, etc.), a 3D graphics core, an audio system, a display driver, a graphics processing unit (GPU), a digital signal processor (DSP), and the like.

Components of the staining intensity detection device 10, such as the processor 100, the RAM 200, the neural network processor 300, the input/output device 400, and the memory 500, may transmit and receive data via a system bus 600. For example, as a standard bus specification, the Advanced Microcontroller Bus Architecture (AMBA) protocol of Advanced RISC Machine (ARM) may be applied to the system bus 600. However, the protocol is not limited thereto, and various types of protocols may be applied.

In one embodiment, components of the staining intensity detection device 10, which are the processor 100, the RAM 200, the neural network processor 300, the input/output device 400, and the memory 500, may be implemented as a single semiconductor chip, for example, the staining intensity detection device 10 may be implemented as a system on a chip (SoC). However, it is not limited thereto, and the staining intensity detection device 10 may be implemented as a plurality of semiconductor chips. In one embodiment, the staining intensity detection device 10 may also be implemented as an application processor mounted on a mobile device.

The processor 100 may control the overall operation of the staining intensity detection device 10, and as an example, the processor 100 may be a central processing unit (CPU). The processor 100 may include a single core or a plurality of cores (multi-core). The processor 100 may process or execute programs and/or data stored in the RAM 200 and the memory 500. For example, the processor 100 may control various functions of the staining intensity detection device 10 by executing programs stored in the memory 500.

The RAM 200 may temporarily store programs, data, or instructions. For example, programs and/or data stored in memory 500 may be temporarily loaded into the RAM 200 according to the control or boot code of the processor 100. The RAM 200 may be implemented using a memory such as a dynamic RAM (DRAM) or static RAM (SRAM).

The input/output device 400 may receive input data from a user or the outside and output a data processing result of the staining intensity detection device 10. The input/output device 400 may be implemented using at least one of a touch screen panel, a keyboard, and various types of sensors. In one embodiment, the input/output device 400 may collect information around the staining intensity detection device 10. For example, the input/output devices 400 may include at least one of various types of sensing devices such as a scanner or may receive a sensing signal from the device. In one embodiment, the input/output device 400 may sense or receive an image signal from the outside of the staining intensity detection device 10 and convert the sensed or received image signal into image data, that is, an image frame. The input/output device 400 may store the image frame in the memory 500 or provide it to the neural network processor 300.

The memory 500 is a storage place for storing data, and it may store, for example, an operating system (OS), various programs, and various types of data. The memory 500 may be a DRAM, but is not limited thereto. The memory 500 may include at least one of a volatile memory and a non-volatile memory. The non-volatile memory may include a read only memory (ROM), a programmable ROM (PROM), an electrically programmable ROM (EPROM), an electrically erasable and programmable ROM (EEPROM), a flash memory, a phase-change RAM (PRAM), a magnetic RAM (MRAM), a resistive RAM (RRAM), a ferroelectric RAM (FRAM), and the like. The volatile memory may include a DRAM, an SRAM, a synchronous DRAM (SDRAM), and the like. In addition, in one embodiment, the memory 500 may be implemented as a storage device such as a hard disk drive (HDD), a solid-state drive (SSD), a compact flash (CF), a secure digital (SD) card, a micro secure digital (micro-SD) card, a mini secure digital (mini-SD) card, an extreme digital (xD) card, or a memory stick.

The neural network processor 300 may generate a neural network model, train a neural network, perform a calculation based on received input data, generate an information signal based on the calculation result, or retrain a neural network. The neural network may include various types of neural network models such as a convolution neural network (CNN), a region with CNN (R-CNN), a region proposal network (RPN), a recurrent neural network (RNN), a stacking-based deep neural network (S-DNN), a state-space dynamic neural network (S-SDNN), a deconvolution network, a deep belief network (DBN), a restricted Boltzmann machine (RBM), a fully convolutional network, a long short-term memory (LSTM) network, and a classification network, but is not limited thereto.

According to one embodiment of the present disclosure, the neural network processor 300 of the staining intensity detection device 10 may train a staining intensity detection model based on input training data and store the trained staining intensity detection model in the memory 500. At this time, the neural network model stored in the memory 500 may be weights and parameters between layers and may be configured in a hierarchical layer structure.

In addition, the neural network processor 300 of the staining intensity detection device 10 may analyze input data input as an analysis target by loading a staining intensity detection model from the memory 500. The neural network processor 300 may output inference data by analyzing an input image based on the neural network model. According to one embodiment, the inference data may include a staining intensity class of each cell included in at least a part of an area in the WSI and a confidence value corresponding to each class.

The processor 100 may identify a class to which each cell belongs among a plurality of staining intensity classes by computing the inference data output from the neural network processor.

According to one embodiment, the processor 100 may adjust a confidence value bias for a pair of classes in response to a user input and re-perform staining intensity extraction for each cell according to the adjusted confidence value bias.

In FIG. 1, the processor 100 and the neural network processor 300 are described as distinct components, but the processor 100 and the neural network processor 300 may be configured as the same hardware and distinguished as software units that perform different functional operations.

Hereinafter, the invention of the present disclosure will be described with reference to FIG. 1.

FIG. 2 shows a diagram illustrating quantitative results calculated according to one embodiment.

Referring to FIG. 2, an image analysis device may analyze a slide image in which a specific cell tissue is stained through a neural network model and may interpret the analytical results to calculate quantitative results. For example, when the image analysis device analyzed a slide image stained with ER/PR/HER2 through the IHC method, ER was expressed at 97% and evaluated as positive, and PR was expressed at 91% and evaluated as positive.

On the contrary, HER2 was expressed at 44% and evaluated as negative, but since the expression level of HER2 is within the error range for evaluating positive and negative, there may be a situation where an evaluation by a pathologist is required in addition to the artificial intelligence (AI) analysis results. In other words, a pathologist may directly analyze the staining intensity of cells on the slide image and determine whether the expression level of a specific protein is dangerous based on the staining intensity.

When the expression level of a specific protein is within the error range and the AI analysis results are inaccurate, there is a high possibility that a pathologist's judgment is also inaccurate. At this time, there may be situations where a specific area within a slide image needs to be carefully examined or the staining intensity of cells needs to be analyzed one by one. n this way, when a pathologist analyzes a stained slide image without any auxiliary devices, not only is it difficult to make an accurate judgment, but the analysis inevitably takes a lot of time.

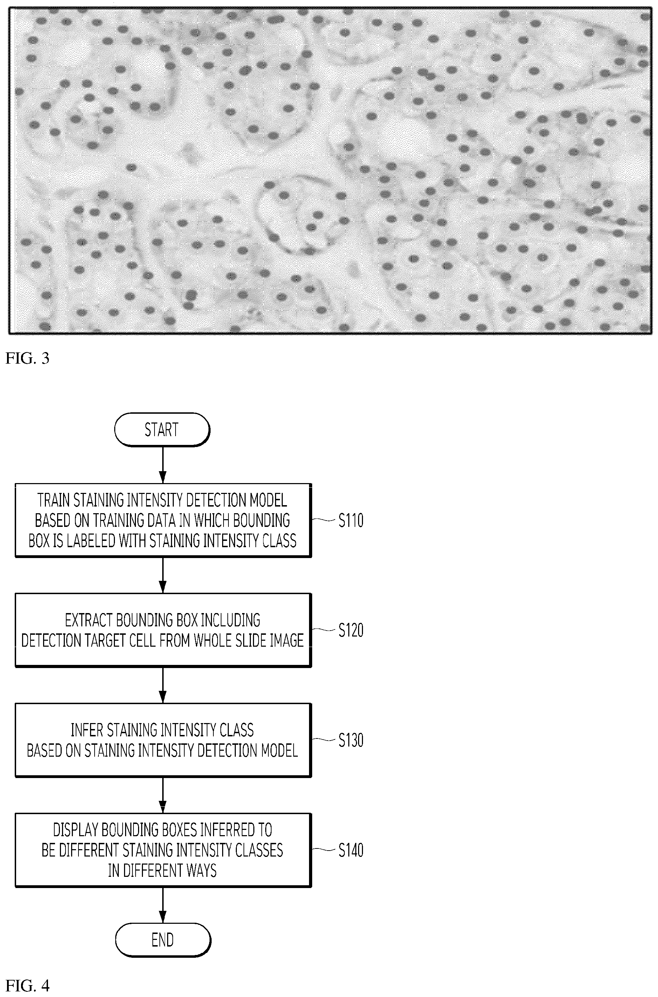

FIG. 3 shows a diagram illustrating an example of detecting cell-by-cell staining intensity according to a previous embodiment.

Referring to FIG. 3, the previous embodiment was an auxiliary device that assisted a pathologist in analysis, by displaying stained cells one by one so that the pathologist may specify the location of the cell to be analyzed.

The previous embodiment may only detect whether a cell is stained using a neural network model, and even when classifying detected cells into several groups according to staining intensity, the classification is performed only through comparison of the RGB values of the stained portion. This method of classifying cells into several groups by comparing the RGB values of the stained portion or comparing the staining intensity with reference values has a problem in that it lacks consistency and thus prevents a precise analysis.

In particular, cells with weak or moderate staining intensity may not be clearly distinguished, which may lead to the risk of missing important diagnostic information in the case of tumor cells.

FIG. 4 shows a flowchart illustrating a staining intensity detection method according to one embodiment.

Referring to FIG. 4, the staining intensity detection device 10 may classify cells with different staining intensity classes by extracting a bounding box including a detection target cell and displaying the bounding box in different ways according to the staining intensity class of the bounding box. The detection target cell may refer to a cell that is a target of staining, such as a tumor cell, and the staining intensity detection device 10 may analyze all detection target cells included in an analysis target area using the bounding box. The bounding box may refer to an image of a specific shape and size with a clearly distinguished boundary, including one detection target cell.

In Step S110, the staining intensity detection device 10 may train a staining intensity detection model based on a training data set in which a bounding box including a detection target cell and a staining intensity class of the detection target cell are labeled.

The training data set may be data in which a bounding box surrounding one detection target cell in a tissue slide image and a staining intensity class of the detection target cell are labeled. The staining intensity class may include a negative tumor cell class, a weak tumor cell class, a moderate tumor cell class, and a strong tumor cell class. The staining intensity class may be a class determined by classifying the degree to which cells that are staining targets, such as ER/PR/HER2, are stained.

According to one embodiment, a bounding box including materials other than a detection target cell may be produced as a training data set without being labeled with a staining intensity class. At this time, a bounding box including materials other than a detection target cell may be a bounding box that does not include any detection target cells.

All bounding boxes classified into staining intensity classes may be displayed on a tissue slide image. In contrast, bounding boxes that are not classified into staining intensity classes may be bounding boxes that are not displayed on a tissue slide image.

Materials other than a detection target cell may include extracellular matrix and may be classified as a non-tumor cell. In addition, non-tumor cells may further include myoepithelial cells, inflammatory cells, endothelial cells, pericytic cells, stromal cells, necrotic debris, and apoptotic bodies.

Inflammatory cells may include lymphocytes, neutrophils, and plasma cells. Stromal cells may include inactive fibroblasts, fibroblasts, and myofibroblasts.

In Step S120, the staining intensity detection device 10 may extract a bounding box including a detection target cell from a WSI. A user may designate an area in the WSI from which the staining intensity of the detection target cells is to be extracted, and the staining intensity detection device 10 may extract bounding boxes including the detection target cells in the area.

According to one embodiment, the staining intensity detection device 10 may extract bounding boxes of the detection target cells based on the shape of the cells regardless of the staining intensity of the detection target cells.

According to another embodiment, the staining intensity detection device 10 may extract bounding boxes classified into each of a plurality of staining intensity classes using a staining intensity detection model. For example, the staining intensity detection device 10 may extract bounding boxes classified into a first staining intensity class and may extract bounding boxes classified into a second staining intensity class. At this time, the bounding boxes classified into the first staining intensity class or the second staining intensity class may be bounding boxes inferred to be the first staining intensity class or the second staining intensity class by a staining intensity detection model with a certain confidence value or higher. Bounding boxes corresponding to each of the first staining intensity class and the second staining intensity class may be extracted independently, and bounding boxes classified into the first staining intensity class by the staining intensity detection model may also be extracted as bounding boxes classified into the second staining intensity class.

In Step S130, the staining intensity detection device 10 may infer the staining intensity class of each bounding box based on the staining intensity detection model. The staining intensity detection model may calculate a confidence value at which the bounding box corresponds to each of the plurality of staining intensity classes. The confidence value may refer to a probability value at which the bounding box corresponds to the corresponding staining intensity class. The staining intensity detection device 10 may infer the bounding box as one of the plurality of staining intensity classes based on the calculated confidence value.

For example, the staining intensity detection device 10 may calculate a confidence value at which the bounding box corresponds to each of the first to fourth staining intensity classes. The staining intensity detection device 10 may infer the staining intensity class having the highest confidence value among the first to fourth staining intensity classes as the staining intensity class of the corresponding bounding box.

In Step S140, the staining intensity detection device 10 may display the bounding box inferred as different staining intensity classes in different ways. In other words, the staining intensity detection device 10 may display the bounding box in different display methods for each staining intensity class. Different methods may mean displaying them in different colors.

The staining intensity detection device 10 may display bounding boxes corresponding to the staining intensity classes in different methods, so that a pathologist analyzing a tissue slide image may confirm whether cells having a certain staining intensity class are identified in an image area to be analyzed.

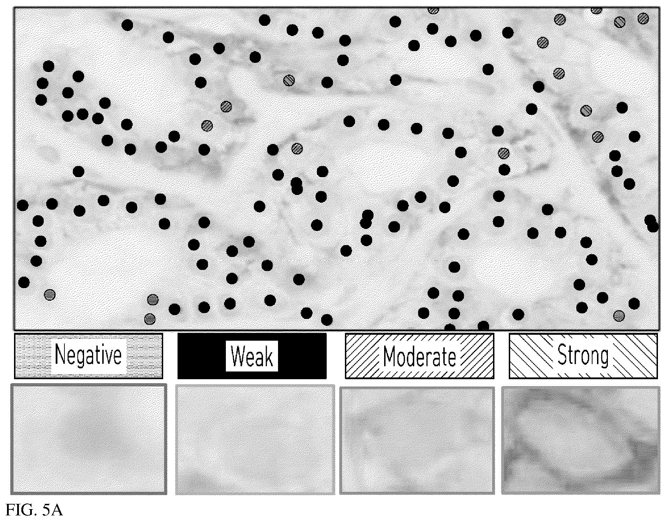

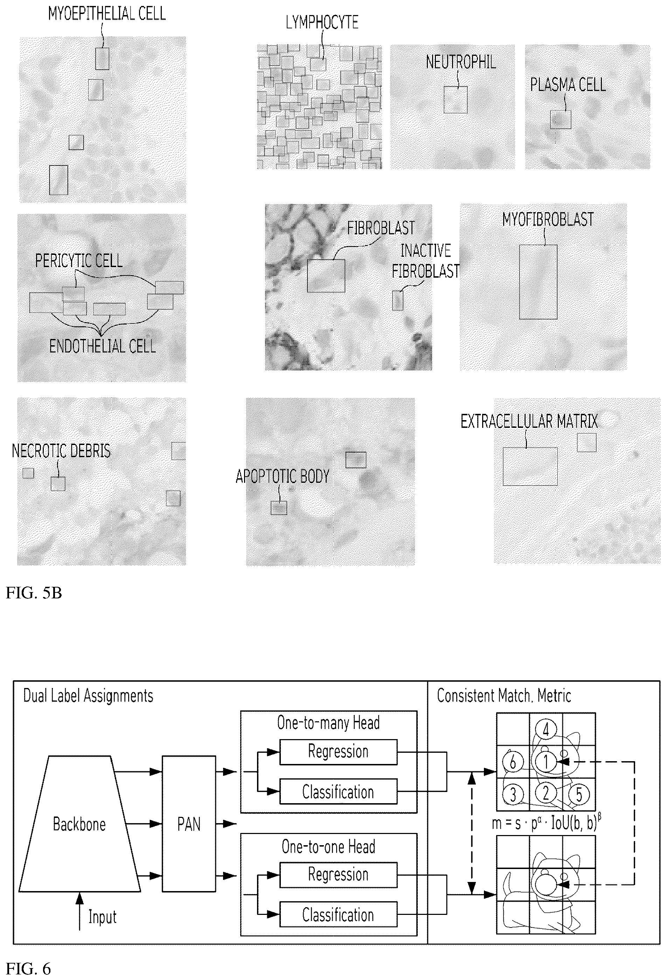

FIGS. 5A and 5B show diagrams illustrating types of training data sets to be input to a staining intensity detection model according to one embodiment.

Referring to FIG. 5A, the training data set to be input to the staining intensity detection model may be a data set in which each bounding box including a detection target cell is labeled with one staining intensity class. The staining intensity class may include a negative tumor cell class, a weak tumor cell class, a moderate tumor cell class, and a strong tumor cell class. The staining intensity class may be a class classified according to the shape, clarity, RGB value, and the like of the detection target cell within the bounding box. The closer the staining intensity class is to a strong tumor cell, the more likely it is that a specific protein to be detected is expressed at a high level in the tissue or cell.

Referring to FIG. 5B, a bounding box that does not include a detection target cell within the bounding box may be prepared as training data. Bounding boxes including materials other than a detection target cell may be generated as a training data set by being labeled with a type other than a staining intensity class, rather than being labeled with a staining intensity class. For example, a type other than a staining intensity class may be referred to as a non-tumor cell.

Non-tumor cells may further include myoepithelial cells, inflammatory cells, endothelial cells, pericytic cells, stromal cells, necrotic debris, apoptotic bodies, and extracellular matrix.

Inflammatory cells may include lymphocytes, neutrophils, and plasma cells. Stromal cells may include inactive fibroblasts, fibroblasts, and myofibroblasts.

All bounding boxes classified into staining intensity classes may be displayed on a tissue slide image. In contrast, bounding boxes that are not classified into staining intensity classes may be bounding boxes that are not displayed on a tissue slide image.

The staining intensity detection model may be trained not only with bounding boxes labeled with staining intensity classes but also with bounding boxes corresponding to non-tumor cells. While the previous neural network model for extracting stained cells was trained only with bounding boxes including stained cells, the staining intensity detection model of the present disclosure is trained with bounding boxes that do not include detection target cells, thereby extracting bounding boxes including detection target cells more accurately.

The trained staining intensity detection model may calculate a confidence value only for bounding boxes corresponding to staining intensity classes and display the bounding boxes in a separate display method. In contrast, the staining intensity detection model does not calculate a confidence value for classifying bounding boxes as non-tumor cells, and it may maintain the original state without separately displaying the bounding boxes.

Specifically, the trained staining intensity detection model may calculate a confidence value that a bounding box corresponds to each of the negative tumor cell class, the weak tumor cell class, the moderate tumor cell class, and the strong tumor cell class, and it may not calculate a confidence value that the bounding box is classified as a non-tumor cell.

It is described that the HER2 biomarker classified into four classes, but the number and types of staining intensity classes according to the embodiment of the present disclosure may not be limited thereto. For example, a biomarker such as Ki-67 may be classified into two classes (negative tumor cell class and positive tumor cell class) excluding a non-tumor cell.

FIG. 6 shows a diagram illustrating a structure of a staining intensity detection model according to one embodiment.

Referring to FIG. 6, the staining intensity detection model may process dual label assignments for input data and output inference results using consistent match metrics.

The backbone network into which input data is input may be a network utilizing a self-supervised learning model based on DIstillation with NO labels (DINO). Through the self-supervised learning model, the backbone network may learn hidden patterns and structures related to a detection target cell within a bounding box on its own. The DINO model may be effectively used to learn visual features within an image in combination with the Vision Transformer architecture.

The staining intensity detection model may utilize a regression model and a classification model in the process of processing dual label assignments. The one-to-many head model may predict a plurality of staining intensity classes for one input bounding box. At this time, the classification model of the staining intensity detection model may classify the staining intensity class of the bounding box, and the regression model may calculate a confidence value for each staining intensity class. In other words, the one-to-many head model may calculate a confidence value for each of the staining intensity classes. In contrast, the one-to-one head model may predict a single staining intensity class for a single input bounding box.

The staining intensity detection model may evaluate consistency by comparing the results of the one-to-many head model with the results of the one-to-one head model through consistent match metrics. Through the consistency evaluation, the confidence value for a bounding box to correspond to each staining intensity class may be accurately calculated.

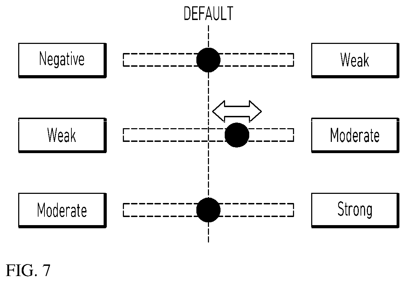

FIG. 7 shows a diagram illustrating a user interface/user experience (UI/UX) capable of adjusting a bias of a confidence value according to one embodiment.

Referring to FIG. 7, the staining intensity detection device 10 may adjust a bias of a confidence value for each pair of staining intensity classes. The staining intensity detection device 10 may have a bias adjustment graphical user interface (GUI) that may be operated by a user, and as the bias adjustment GUI is adjusted, the parameters of the staining intensity detection model may be adjusted so that staining intensity class re-detection is performed for the bounding box.

According to one embodiment, the confidence value for a staining intensity class for which adjustment is input may be adjusted by assigning a weight or penalty to a loss function of the staining intensity detection model. For example, when a bias adjustment between a second staining intensity class and a third staining intensity class is input, the staining intensity detection model may adjust the weight of the loss function corresponding to the staining intensity detection model.

When a bias adjustment GUI between the weak tumor cell class and the moderate tumor cell class is shifted to the right, the confidence value may be adjusted so that more bounding boxes corresponding to the moderate tumor cell class can be detected. When the staining intensity detection model receives an input in which the bias adjustment GUI is shifted to the right, the loss function value corresponding to the moderate tumor cell class may be adjusted to assign a high confidence value to bounding boxes with relatively low similarity. This may increase the probability that the bounding boxes are classified into the moderate tumor cell class.

When the staining intensity detection model receives an input in which the bias adjustment GUI between the weak tumor cell class and the moderate tumor cell class is shifted to the left, the loss function value corresponding to at least one class of the weak tumor cell class and the moderate tumor cell class may be adjusted to assign a low confidence value to bounding boxes with relatively high similarity. This may decrease the probability that the bounding boxes are classified into the moderate tumor cell class.

The staining intensity of a tissue slide image may vary depending on the performance of the scanner and the staining situation. Therefore, when a pathologist determines that it is necessary to adjust the confidence value bias of a certain staining intensity class for a stained tissue slide image, the bias may be easily adjusted through a GUI according to one embodiment of the present disclosure.

The bias adjustment GUI according to one embodiment of the present disclosure may be formed horizontally for each pair of staining intensity classes so that the bias may be adjusted through left-and-right scrolling but is not limited thereto, and it may also be formed vertically so that the bias may be adjusted through up-and-down scrolling. Furthermore, the bias adjustment GUI of the present disclosure may adjust the bias of a staining intensity class not only through scrolling in a certain direction but also through all kinds of GUIs capable of adjustment in a stepwise manner.

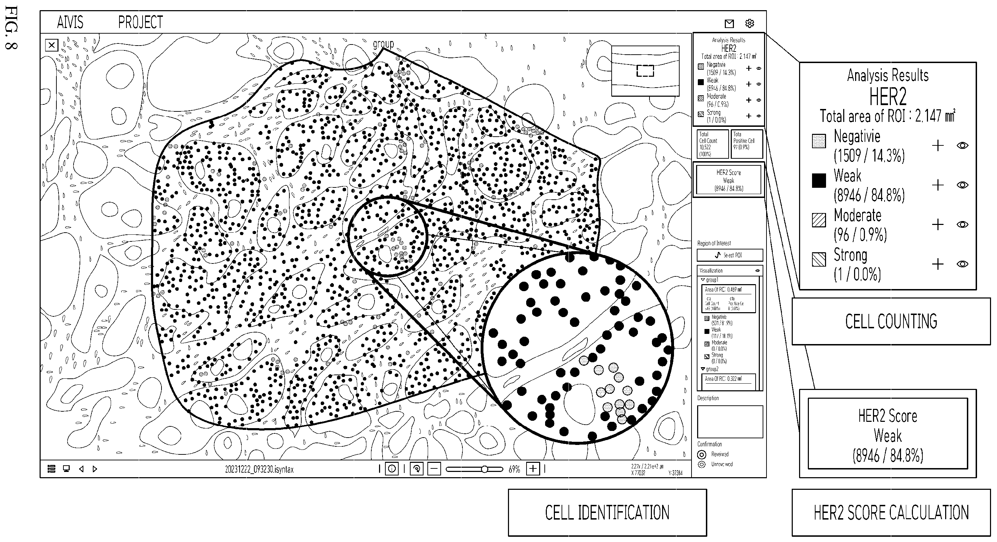

FIG. 8 shows a diagram illustrating an example of displaying cell-by-cell staining intensity according to staining intensity class according to one embodiment.

Referring to FIG. 8, the staining intensity detection device 10 according to one embodiment of the present disclosure may identify an area in which staining intensity of a detection target cell is to be detected by a user's drag input. The staining intensity detection device 10 may extract bounding boxes including detection target cells from the identified area and classify the staining intensity class for each bounding box.

The staining intensity detection device 10 may count the number of bounding boxes for each staining intensity class within the identified area. The number of counted bounding boxes may be displayed on a part of the display of the staining intensity detection device 10, and the percentage of the number of bounding boxes for each staining intensity class based on the total number of bounding boxes may also be displayed.

The staining intensity detection device 10 may calculate a final score based on the percentage calculated for each staining intensity class. The final score may be expressed as a staining intensity class of the identified area. The staining intensity class may indicate the degree of a certain protein expressed within a cell, and the degree to which a certain protein is expressed in the area may be calculated as the final score.

According to the embodiment shown in FIG. 8, the staining intensity detection device 10 may detect the staining intensity for the HER2 protein. The number of bounding boxes classified into the negative tumor cell class by the staining intensity detection model in the areas dragged by the user may be 1509, and the number of bounding boxes classified into the weak tumor cell class may be 8946. In addition, the number of bounding boxes classified into the moderate tumor cell class may be 96, and the number of bounding boxes classified into the strong tumor cell class may be 1.

The staining intensity detection device 10 may calculate the percentage of the number of bounding boxes for each staining intensity class based on the total number of bounding boxes. The percentage for the negative tumor cell class may be 14.3%, the percentage for the weak tumor cell class may be 84.8%, the percentage for the moderate tumor cell class may be 0.9%, and the percentage for the strong tumor cell class may be 0.0%.

The staining intensity detection device 10 may calculate the staining intensity class with the highest percentage as the final score. Since the percentage for the weak tumor cell class is 84.8%, the staining intensity detection device 10 may determine the final staining intensity score of the dragged area as weak.

The staining intensity detection device 10 according to one embodiment of the present disclosure can quickly and accurately provide information on the staining intensity class with which detection target cells are stained in an area that a pathologist wants to examine. In addition, each staining intensity class may be displayed in a different display method, and a pathologist may intuitively confirm where the target cells classified into a strong staining intensity class are located. In other words, in the past, pathologists had no choice but to examine where the target cells strongly stained were located one by one, which was time-consuming or sometimes resulted in the failure to find the detection target cells. In contrast, the staining intensity detection device 10 of the present disclosure enable pathologists to immediately identify target cells with risk factors through an intuitive display method (e.g., displaying in red).

Meanwhile, the methods according to the various embodiments of the present invention described above may be implemented in the form of an application or software program that may be installed on an existing electronic device.

In addition, all or part of the method may be configured as several software function modules and implemented in an operating system (OS). Alternatively, each step may be configured as one software function module, or individual steps may be combined to be configured as one software function module and implemented on an OS. Therefore, even when some embodiments of the present disclosure are not implemented entirely as one software function module, when several software function modules implement each step of the present disclosure and several software function modules are implemented in one OS, it may be understood that the method of the present disclosure is implemented.

In addition, the methods according to the various embodiments of the present invention described above may be implemented even only with a software upgrade or a hardware upgrade for an existing electronic device. In addition, it is also possible that the various embodiments of the present invention described above are performed through an embedded server installed in an electronic device or an external server of the electronic device.

Meanwhile, according to one embodiment of the present invention, the various embodiments described above may be implemented as software including instructions stored in a computer readable recording medium that may be read by a computer or a similar device using software, hardware, or a combination thereof. In some cases, the embodiments described herein may be implemented as a processor itself. According to a software implementation, embodiments such as the procedures and functions described herein may be implemented as separate software modules. Each of the software modules may perform one or more functions and operations described herein.

Meanwhile, a computer or a similar device may include a device according to the disclosed embodiments, which is a device capable of calling stored instructions from a storage medium and operating according to the called instructions. When the instructions are executed by a processor, the processor may directly or perform functions corresponding to the instructions by using other components under the control of the processor. The instructions may include code generated or executed by a compiler or an interpreter.

A recording medium that may be read by a device may be provided in the form of a non-transitory computer readable recording medium. Here, the term 'non-transitory' means that the storage medium includes no signal and is tangible, but does not distinguish whether data is stored semi-permanently or temporarily in the storage medium. At this time, the non-transitory computer readable medium refers to a medium that semi-permanently stores data and may be read by a device, rather than a medium that stores data for a short moment, such as a register, cache, or memory. Specific examples of non-transitory computer readable media may include CDs, DVDs, hard disks, Blu-ray disks, USBs, memory cards, ROMs, and the like.

The method of detecting staining intensity according to one embodiment of the present disclosure can detect the cell-by-cell staining intensity of stained tumor cells in a WSI, thereby performing accurate staining intensity detection. In addition, by visualizing the staining intensity into a plurality of classes as well as the presence or absence of staining, the method can enable pathologists to easily identify the staining intensity.

The effects that may be obtained from the exemplary embodiments of the present disclosure are not limited to the above-mentioned effects, and other effects that are not mentioned may be clearly derived and understood by one of ordinary skill in the technical field to which the exemplary embodiments of the present disclosure pertain, from the following description. In other words, unintended effects resulting from implementing the exemplary embodiments of the present disclosure may also be derived by one of ordinary skill in the technical field from the exemplary embodiments of the present disclosure.

As described above, exemplary embodiments are disclosed in the drawings and the specification. Although specific terms have been used herein to describe the embodiments, these have been used only for the purpose of explaining the technical idea of the present disclosure and have not been used to limit the meaning or the scope of the present disclosure described in the claims. Therefore, those skilled in the art will understand that various modifications and other equivalent embodiments are possible therefrom. Accordingly, the genuine technical protection scope of the present disclosure should be determined by the technical idea of the appended claims.

## Claims

1. A method of detecting cell-by-cell staining intensity from a pathological image composed of a tissue slide image, the method comprising:
extracting a bounding box including a detection target cell from the tissue slide image;
inferring a staining intensity class of the bounding box based on a trained staining intensity detection model; and
displaying bounding boxes inferred to be different staining intensity classes in different ways.

2. The method of claim 1, wherein the staining intensity classes are classified into a negative tumor cell class, a weak tumor cell class, a moderate tumor cell class, and a strong tumor cell class.

3. The method of claim 2, further comprising: training the staining intensity detection model based on a training data set labeled with one of a plurality of staining intensity classes for the bounding box.

4. The method of claim 3, wherein the staining intensity detection model is trained based on a training data set labeled as a non-tumor cell whose bounding box is not included in the staining intensity class.

5. The method of claim 1, wherein the step of inferring the staining intensity class includes:
calculating a confidence value for each staining intensity class for the bounding box; and
selecting one of the staining intensity classes among the plurality of staining intensity classes based on the calculated confidence value.

6. The method of claim 5, wherein the confidence value has a bias that is adjustable by a user for each pair of the staining intensity classes, and when at least one bias is adjusted, reselection of the staining intensity class is performed.

7. The method of claim 1, wherein the staining intensity detection model is a model utilizing a self-supervised learning-based feature extraction model as a backbone network.
